# EUROPEAN PATENT SPECIFICATION

(11) **EP 1 333 846 B1**
(45) Date of publication and mention of the grant of the patent: **18.04.2012**
(21) Application number: 01979134.2
(22) Date of filing: 16.10.2001
(51) Int. Cl.: A61K 35/39, A61P 3/10

(54) **PREPARATION AND XENOTRANSPLANTATION OR PORCINE ISLETS**
ZUBEREITUNG UND XENOTRANSPLANTATION VON LANGERHANS-INSELN VON SCHWEINEN
PREPARATION ET XENOGREFFE D'ILOTS PORCINS

(30) Priority: 17.10.2000 NZ 50761600; 02.11.2000 NZ 50796300
(43) Date of publication of application: 13.08.2003
(73) Proprietor: Diabcell Pty Limited, Sydney NSW 2001 (AU)
(72) Inventor: ELLIOTT, Professor Robert Bartlet, Papatoetoe, Auckland (NZ); BASTA, Guiseppe, University of Perugia, I-06126 Perugia (IT); LUCA, Giovanni, University of Perugia, I-06126 Perugia (IT); CALAFIORE, Riccardo, University of Perugia, I-06126 Perugia (IT)
(74) Representative: Wright, Simon Mark
(86) International application number: PCT/NZ2001/000228
(87) International publication number: WO 2002/032437

(56) References cited:
- WO-A-97/39107
- WO-A1-01/52871
- AU-A- 8 186 498
- US-A- 5 849 285
- US-A- 6 146 653
- LONDON N.J. ET AL: 'A simple method for the release of islets by controlled collagenase digestion of the human pancreas' TRANSPLANTATION vol. 49, no. 6, June 1990, pages 1109 - 1113, XP002959409
- SELAWRY H.P. ET AL: 'Sertoli cell-enriched fractions in successful islet cell transplantation' CELL TRANSPLANTATION vol. 2, 1993, pages 123 - 129, XP000602285
- KORBUTT G.S. ET AL: 'Cotransplantation of allogenic islets with allogenic testicular cell aggregates allows long-term graft survival without systemic immunosuppression' DIABETES vol. 46, February 1997, pages 317 - 322, XP002962502
- RAYAT G.R. ET AL: 'Potential application of neonatal porcine islets as treatment for type 1 diabetes: a review' ANNALS OF THE NEW YORK ACADEMY OF SCIENCES vol. 875, 18 June 1999, pages 175 - 188, XP002962522
- SUAREZ-PINZON W. ET AL: 'Testicular sertoli cells protect islet beta-cells from autoimmune destruction in NOD mice by a transforming growth factor-beta1-dependent mechanism' DIABETES vol. 49, November 2000, pages 1810 - 1818, XP002968228
- LUCA G. ET AL: 'Sertoli cell-induced reversal of adult rat pancreatic islet beta-cells into fetal-like status: potential implications for islet transplantation in type 1 diabetes mellitus' JOURNAL OF INVESTIG. MEDICINE vol. 48, no. 6, November 2000, pages 441 - 448, XP002962531
- SELAWRY H.P. ET AL: 'Sertoli cell-induced effects on functional and structual characteristics of silated neonatal porcine islets' CELL TRANSPLANTATION vol. 5, no. 5, 1996, pages 517 - 524, XP002040065
- CALAFIORE R. ET AL: 'Transplantation of pancreatic islets contained in minimal volume microcapsules in diabetic high mammalians' ANNALS OF THE NEW YORK ACADEMY OF SCIENCES vol. 875, 1999, pages 219 - 232, XP002957509
- HYDER ET AL: "Effect of the pancreatic digestion with liberase versus collagenase on the yield, function and viability of neonatal rat pancreatic islets", CELL BIOLOGY INTERNATIONAL, ACADEMIC PRESS, GB LNKD- DOI:10.1016/J.CELLBI.2005.05.004, vol. 29, no. 9, 1 September 2005 (2005-09-01), pages 831-834, XP005051934, ISSN: 1065-6995

## Description

The present invention relates to improvements in and/or relating to the treatment of diabetes using xenotransplantation. More particularly but not exclusively the present invention relates to the preparation of viable xenotransplantable porcine islets and/or the treatment of a mammalian patient (including humans) suffering from diabetes involving the transplantation into the mammal of viable porcine islets capable of producing insulin within the host and the associated use of sertoli cells within the procedure.

### Background

Type 1 (insulin-dependent) diabetes mellitus is a common endocrine disorder that results in substantial morbidity and mortality, and leads to considerable financial costs to individual patients and healthcare systems.

Treatment with insulin, while life-saving, often does not provide sufficient control of blood glucose to prevent the feared complications of the disease, which has provided the impetus for intensive research into better methods of sustaining normoglycaemia.

Among the newer treatment strategies that have been proposed, transplantation of pancreatic islet cells, obtained either from other humans or animals, has received the most attention worldwide. This is because transplantation can restore not only the insulin-secreting unit, but also the precise fine tuning of insulin release in response to multiple neural and humoral signals arising within and beyond the islets of Langerhans.

Human islet cell transplantation is limited by the shortage of human islet tissue. The use of pig islet cells is currently viewed as the most promising alternative since:
I. the supply of pig cells can be readily expanded by optimising the supply of donor animals;
II. pig and human insulin have close structural similarities; and
III. physiological glucose levels in pigs are similar to those in humans.

The rationale for this treatment approach (termed 'xenotransplantation') is that the implanted pig islets have the potential to mimic the normal physiological insulin response in type 1 diabetics such that near-normal blood glucose levels may be achievable without insulin administration or with a reduced requirement for it. As a consequence, long-term diabetes complications may be prevented and patients should experience less hypoglycaemia than they do with the currently recommended 'intensive' insulin regimens.

Allograft (same species transplantation) of pancreatic islets, separated from human donor organs, could induce full remission of hyperglycaemia and discontinuation of exogenous insulin treatment in patients with Type 1 diabetes mellitus. However, full success of this approach is still hampered by several problems, and in particular:
a) islet disconnection from the native pancreas interrupts a fine neuro-and microcapillary vascular, as well as humoral regulatory network which might cause ischemia, apoptosis and release of mediators of the inflammatory reaction;
b) Isolated islets are very vulnerable to adverse immunologic and environmental conditions.

Although immune rejection is a major problem for transplantation, problems with longevity of the islet transplant unrelated to the immune system may also prevent successful islet cell transplantation. This may be ascribed to unfavourable local environmental conditions because of the heterotopic nature of the transplant site's anatomy (usually the liver) which greatly differs from the native pancreas, non specific mediators of the inflammation,-cell-induced apoptosis, or other unknown factors. Nevertheless, the extremely slow -cell turnover significantly contributes to abbreviate the functional life-span of such a differentiated tissue. Hence attempts to expand the original grafted -cell mass should be implemented.

Previous studies reporting on induction of insulin release from whole islets, which had been exposed to hormones, such as prolactin, human placental lactogen, and growth hormone, failed to demonstrate significant increase in the islet -cell replication rate¹, thereby clouding the interpretation about the origin and mechanisms of the increased insulin secretion. In subsequent studies, adult human islet -cells were shown to expand during in vitro culture maintenance, within special artificial tissue matrices, and in presence of growth factors². However, in this particular instance, the islets had been dispersed into single cells and cultured as cell monolayers. Moreover, there was no significant correlation between the increased -cell mitotic index and increase in insulin concentration. Last, the impact of islet cell aggregates, rather than whole islets, on transplantation remains unpredictable and it has not been further pursued.

In our previous work, particularly as detailed in PCT/NZO 1/0009 we have disclosed the preparation and use of xenotransplantable porcine islets for the treatment of diabetes.

Selawry et al. (Cell Transplant; 5:517-524 (1996) describes Sertoli -cell induced defects on functional and structural characteristics of isolated neonatal porcine islets. US 5,849,285 describes antoimmune disease treatment with Sertoli cells and *in vitro* co-culture of mammal cells with Sertoli cells.

### Object

It is an object of the present invention to provide an improved method of preparing porcine islets which produces islets viable for xenotransplantation into a mammalian patient the islets being capable of producing insulin within a mammalian host, as well as the islet preparation so produced, or irrespectively or how produced, or a similar form.

Alternatively or additionally, it is a further object to provide an improved method of treating a mammalian patient suffering from diabetes which involves the xenotransplantation of porcine islets into the mammalian patient.

Alternatively or additionally, it is a further object to at least provide the public or medical community with a useful alternative approach to diabetes treatment.

### Statements of Invention

In a first aspect the invention consists in **a method of preparing a xenotransplantable porcine islet preparation capable upon xenotransplantation of producing porcine insulin in an appropriate recipient mammal**, the method including or comprising:
(i) harvesting the pancreas of piglets at or near full term gestation, and
(ii) extracting islets from a culture of the harvested pancreas using a suitable collagenase,
(iii) the culture of the harvested pancreas being -
   a) of mechanically reduced harvested pancreas, and
   b) a supportive mammalian albumin substantially free of non-human microbiological agents, wherein the pancreas and/or islets are subject to the trauma protecing agent Lignocaine, and wherein the islets are mixed with and/or co-cultured with Sertoli cells.

Preferably the islets (at least at some stage in the performance of the method) are exposed to nicotinamide.

The piglets are at from -20 to +20 days of full term gestation.

Preferably the piglets are at-7 to +10 days of full term gestation.

Preferably the mammalian albumin is human serum albumin (HSA).

Preferably the collagenase is selected from human Liberase® and porcine Liberase@. Preferably said Liberase ® is human Liberase ®.

Preferably the islets are treated with nicotinamide after their extraction from the pancreas.

Preferably the method includes the further step of treating the islets with IgF-1 or the N-terminal tripeptide of IgF-1 (GPE).

Preferably preparation or isolation of the Sertoli cells include use of, or exposure to a buffer to selectively reduce germinal cells.

Preferably the buffer is tris-(hydroxymethyl)-aminomethane hydrochloride.

Preferably the ratio of Sertoli cell to islets employed is substantially 10-1,000 Sertoli cells/islet.

In another aspect the invention consists **in a xenotransplantable porcine islet preparation** prepared according to the above method.

Also described herein is **a method of preparing an implantable device which includes at least one xenotransplantable porcine islet capable upon xenotransplantation of producing porcine insulin in an appropriate recipient mammal**, said method including all comprising:
(i) harvesting the pancreas of a piglet or piglets at -20 to +20 days of full term gestation,
(ii) mechanically reducing the harvested pancreas in the presence of an islet trauma protecting agent and producing a culture of the mechanically reduced harvested pancreas supported by human serum albumin (HSA),
(iii) isolating the islet cells from the culture of mechanically reduced harvested pancreas using a collagenase selected from human Liberase ® and porcine Liberase ®, and
(iv) incorporation of the islets cells in a device suitable for implantation into a recipient mammal,
wherein nicotinamide is introduced to the islets or islet cells prior to incorporation at any one or more stages of the procedure, and
wherein, at least to the extent required to convert porcine non insulin producing islet cells to porcine insulin producing islet cells, at some stage in the procedure presenting the islets and/or islet cells to IgF-1 or the N-terminal tripeptide of IgF.1 (GPE), and
wherein (at least at some stage in the method) the islet cells are associated with Sertoli cells.

Preferably the Sertoli cells are employed within the device to substantially prevent or reduce tissue immune rejection.

Preferably the Sertoli cells are mixed with the islets, prior to insertion into, or within, the device. Preferably preparation or isolation of the Sertoli cells include use of, or exposure to a buffer to selectively reduce germinal cells.

Preferably the buffer is tris-(hydroxymethyl)-aminomethane hydrochloride.

Preferably the Sertoli cells are co-cultured with the islets prior to incorporation.

Preferably the ratio of Sertoli cell to islets employed is substantially 10-1,000 Sertoli cells/islet. More preferably the Sertoli cells are mixed with the islets substantially in a ratio of 10-1,000 cells/islet.

Preferably an antibiotic is used in the isolation of the islet cells.

Preferably the antibiotic is ciproxin.

Preferably the islet trauma protecting agent is Lignocaine.

The device may be a suitable vascularised subcutaneous collagen tube and one or more islet cells are confined within the tube.

The device may be a capsule formed from a biocompatible xenotransplantable material which, in vivo, is both glucose and insulin porous, and one or more islet cells are encapsulated within the capsule.

Preferably said biocompatible material is a suitable alginate.

Preferably the alginate is in ultra pure form.

Preferably the encapsulation provides a surround which prevents, once implanted, direct tissue contact with the islets.

Preferably each encapsulation involves presenting islets and a suitable alginate solution into a source of compatible cations thereby to entrap the islets in a cation - alginate gel.

Preferably said cation alginate gel is calcium-alginate gel.

Preferably said alginate used in the solution is sodium alginate, and the islets and sodium alginate solution is presented as a droplet into a bath of suitable cations.

Preferably the islets and sodium alginate solution is of 1.6% w/w.

Preferably the islets and sodium alginate solution is presented as a droplet through a droplet generating needle.

Preferably the suitable cations are calcium chloride.

Preferably the gel encased islets are coated with a positively charged material and thereafter are provided with an outer coat of a suitable alginate.

Preferably the positive charging material is poly-L-ornithine.

Preferably the gel entrapping the islets within the outer coating is then liquefied.

Preferably the liquification involves or comes about by the addition of sodium citrate. Preferably the encapsulation produces capsules.

Preferably the capsules contain a plurality of islet cells.

Preferably the capsules contain substantially three islet cells.

Preferably the capsules have a diameter of substantially from about 300 to 400 microns.

In another aspect the present invention comprises an implantable device comprising the xenotransplantable porcine islet preparation of the present invention.

The above device may be a vascularised subcutaneous tube comprising the xenotransplantable porcine islet preparation, which is capable of producing porcine insulin in response to glucose within a recipient mammal,
- the tube in vivo limiting the egress of the islet cells and allowing an abundant access to blood vessels,
- the tube also employing or containing Sertoli cells to substantially reduce or prevent tissue immune rejection within the recipient mammal.

Preferably the Sertoli cells are mixed with the islets prior to insertion into, or within, the tube.

Preferably the Sertoli cells are mixed with the islets substantially in a ratio of 10-1,000 cells/islet.

Preferably said culture has been of mechanically reduced harvested pancreatic tissue, such tissue having been exposed to a trauma reducing agent.

The islet cells may have been isolated from a culture supported by HSA using a suitable Liberase® and prior to encapsulation having been exposed to nicotinamide.

In yet another aspect of the invention, the above implantable device may comprise a **xenotransplantable capsule** comprising the xenotransplantable porcine islet preparation, which is capable of producing porcine insulin in response to glucose within a recipient mammal within its biocompatible encapsulating material or materials, the capsule being such that the encapsulation is such as to prevent tissue contact with said islet cell (s) by tissue of a recipient mammal yet **in vivo** will allow glucose entry to the islet cells and the egress of porcine insulin from such islet cells,
wherein the islet cells are associated with Sertoli cells.

The islet cells may have been isolated from a culture supported by HSA using a suitable Liberase® and prior to encapsulation have been exposed to nicotinamide.

Preferably the culture has been of mechanically reduced harvested pancreatic tissue, such tissue having been exposed to a trauma reducing agent.

Preferably the encapsulation has been of islet cells in the presence of a suitable antibiotic.

Preferably the Sertoli cells are mixed with the islets prior to encapsulation.

Preferably the Sertoli cells are co-cultured with the islet cells prior to encapsulation.

Preferably the Sertoli cells: islet ratio is 10-1,000 cells: islet.

The invention also provides an implantable device of the invention for use in the treatment of a mammalian patient predisposed to or suffering from diabetes.

Further described herein is **a method of treating a mammalian patient predisposed to or suffering from diabetes** which involves the xenotransplantation into such patient at least one vascularised subcutaneous tube, capsule or implant of the present invention.

Additionally described herein is **a method for the treatment of a mammalian patient** suffering from or predisposed to diabetes, said method including or comprising the steps of:
(A)
   (i) harvesting the pancreas of piglets at or near full term gestation,
   (ii) culturing the harvested pancreas in mammalian albumin substantially free of non-human microbiological agents,
   (iii) simultaneously with step (ii) and/or after step (ii), extracting the islets from the
      harvested pancreas using a suitable Liberase ®,
      wherein the islets (at least at some stage in the performance of (A)) are exposed to nicotinamide;
(B)
   (i) incorporation of the islets cells, together with Sertoli cells in a device or vehicle suitable for delivery or implantation or xenotransplantation into the recipient mammal,
   (ii) implanting the islets so incorporated into the recipient mammal.

Preferably the Sertoli cells are mixed with the islets, prior to insertion into, or within, the device.

Preferably the Sertoli cells are mixed with the islets substantially in a ratio of 10-1,000 cells/islet.

Preferably there is included also the step of administering nicotinamide to the recipient mammal prior to or after the implantation step.

Preferably the method further includes the step of prescribing for the patient, prior to or after the implantation step, a casein-free diet (as described herein).

Preferably the method further includes the step of subjecting the patient prior to or after the implantation step to a cholesterol lower drug regime.

Preferably the cholesterol lowering drug is of the "statin" family

Preferably the cholesterol lowering drug is Pravastatin ® or Simvistatin ®.

In one preferred form the device of step (B)(i) is a suitable vascularised subcutaneous collagen tube.

In an alternative preferred form the device of step (B)(i) is a capsule of a biocompatible, xenotransplantable material which is, in vivo, both glucose and insulin porous and one or more islet cells are encapsulated within the capsule.

Preferably the biocompatible xenotransplantable material is a suitable alginate.

Also described herein is **a method of preparing an implant which includes at least one xenotransplantable porcine islet capable upon xenotransplantation of producing porcine insulin in an appropriate recipient mammal** including the steps of:
- preparation of porcine pancreatic islets,
- preparation of Sertoli cells,
- preparation of the implant incorporating the Sertoli cells and porcine islets.

Preferably preparation or isolation of the Sertoli cells include use of, or exposure to a buffer to selectively reduce germinal cells.

Preferably the buffer is tris-(hydroxymethyl)-aminomethane hydrochloride.

In one form the implant is (one or more) capsules within which (one or more) islets are encapsulated.

Preferably the Sertoli cells are encapsulated with the islets.

Preferably the Sertoli cells are co-cultured with the islets prior to encapsulation.

Preferably the ratio of Sertoli cell to islets employed is substantially 10-1,000 Sertoli cells/islet.

Preferably the co-culturing results in induced or increased β-cell proliferation and consequential increased endogenous insulin output.

Preferably the preparation of porcine islets includes the steps of harvesting and extracting the islets.

Preferably the porcine islets are prepared substantially according to one of the methods of preparing a xenotransplantatible porcine islet preparation hereinbefore described.

Preferably the encapsulation regime includes the steps of encapsulating the co-cultured islets and Sertoli cells within a biocompatible xenotransplantable material, the material *in vivo* being both glucose and insulin porous.

Preferably nicotinamide is introduced to the islets or islet cells or co-culture prior to encapsulation at any one or more stages of the procedure.

Preferably, at least to the extent required to convert porcine non insulin producing islet cells to porcine insulin producing islet cells, at some stage in the procedure the islets and/or islet cells are presented to IgF-1 or the N-terminal tripeptide of IgF-1 (GPE).

Preferably an antibiotic is associated with the islet cells.

Preferably said antibiotic is ciproxin.

Preferably said islet trauma protecting agent is lignocaine.

Preferably said biocompatible material is a suitable alginate.

Preferably the alginate is in ultra pure form.

Preferably each islet or grouping of islets is entrapped in an *in vivo* insulin and glucose porous biocompatible alginate or alginate like surround.

Preferably the encapsulation provides a surround which prevents, once implanted, direct tissue contact with the islets.

Preferably each encapsulation involves presenting islets and a suitable alginate solution into a source of compatible cations thereby to entrap the islets in a cation - alginate gel.

Preferably said cation alginate gel is calcium-alginate gel.

Preferably said alginate used in the solution is sodium alginate, and the islets and sodium alginate solution is presented as a droplet into a bath of suitable cations.

Preferably the islets and sodium alginate solution is of 1.6% w/w.

Preferably the islets and sodium alginate solution is presented as a droplet through a droplet generating needle.

Preferably the suitable cations are calcium chloride.

Preferably the gel encased islets are coated with a positively charged material and thereafter are provided with an outer coat of a suitable alginate.

Preferably the positive charging material is poly-L-ornithine.

Preferably the gel entrapping the islets within the outer coating is then liquefied.

Preferably the liquification involves or comes about by the addition of sodium citrate.

Preferably the encapsulation produces capsules.

Preferably the capsules contain a plurality of islet cells.

Preferably the capsules contain substantially three islet cells.

Preferably the capsules have a diameter of substantially from about 300 to 400 microns.

In a second form the implant is a vascularized subcutaneous tube within which (one or more) islets are contained.

Preferably the Sertoli cells are contained with the islets.

Preferably the Sertoli cells are co-cultured with the islets prior to encapsulation.

Preferably the ratio of Sertoli cell to islets employed is substantially 10-1,000 Sertoli cells/islet. Preferably the co-culturing results in induced or increased β-cell proliferation and consequential increased endogenous insulin output.

### Definitions

As used herein:
- *"Administering"* includes self-administering;
- *"Casein-free"* when referring to milk as used herein refers to milk which does not contain a diabetogenic factor, particularly to milk containing no variant of -casein which stimulates diabetogenic activity in humans. With reference to International PCT Application WO 96/14577, a non-diabetogenic variant for example, may be the A2 variant of -casein. The full contents of PCT/NZ95/00114 (WO 96/14577) and PCT/NZ96/00039 (WO 96/36239) are relevant by way of reference.
- *"Casein-free"* as used herein in respect of dietary considerations means at least a substantial avoidance (preferably total avoidance) of such milk containing or derived diabetogenic factors.
- The *N-terminal tripeplide of Ig F-1 or "GPE"* is a tripelide (gly-pro-glu) derived from Ig F-1
- *"mammalian albumin* " as used herein preferably means human serum albumin
- *"appropriate "Collagenase"* or *"Liberase* ®" preferably means Liberase H ®
- *"mechanically reduced* " as used herein includes any process where pancreatic tissue is increased in surface area eg, mechanical or water jet shredding, grinding, mincing, etc...
- *"Sertoli cells* " Sertoli cells are derived from the testes of the donor male piglets.
- This class of cell secretes substances which produce immune tolerance. The exact mechanism is not known, but it has been suggested that growth factors such as Tissue Transforming Factor may be responsible for the desired effect.

This invention may also be said broadly to consist in the parts, elements and features referred to or indicated in the specification of the application, individually or collectively, and any or all combinations of any two or more of said parts, elements or features, and where specific integers are mentioned herein which have known equivalents in the art to which this invention relates, such known equivalents are deemed to be incorporated herein as if individually set forth.

The invention consists in the foregoing and also envisages constructions of which the following gives examples.

### DESCRIPTION OF THE DRAWINGS

Preferred forms of the present invention will now be described with reference to the accompanying drawings in which:
- **Figure 1**: shows a preferred procedure for harvesting, isolating and preparing islet cells (with either confinement or encapsulation) and the associated treatment regime for a diabetic human patient in order to receive ongoing benefit from the xenotransplantation,

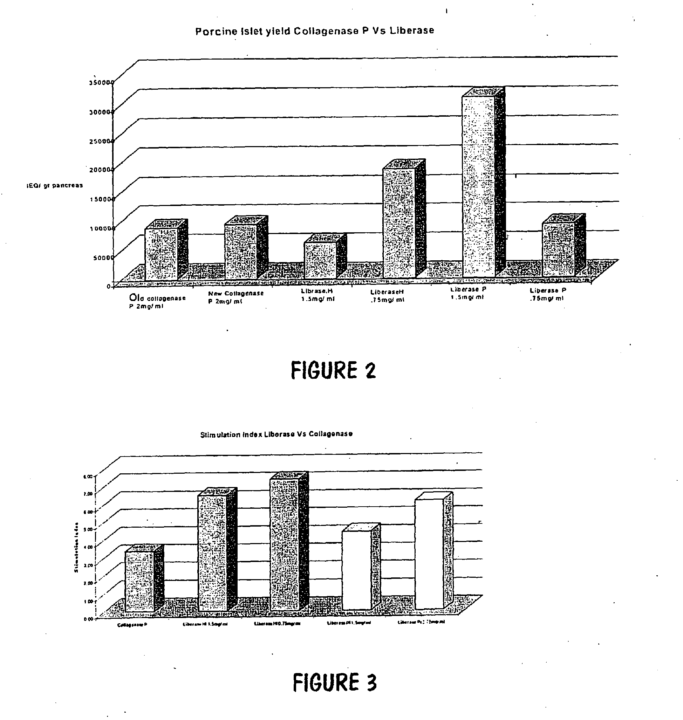

- **Figure 2**: shows the effect of collagenase from various sources on islet yield and function,
- **Figure 3**: shows the stimulation index of Liberase™ against Collagenase clearly showing that Liberase™ preparations (both human and porcine at suitable concentrations) gave higher yields and function in vitro than an optimised concentration of Collagenase P,
- **Figure 4**: shows the stimulation index of free islets when comparing the use of ciproxin against a penicillin/streptomycin mix and against a control of no antibiotics,
- **Figure 5**: shows a plot of confined piglet islets (eg; using a vascularised subcutaneous tube collagen tube) and the post transplant relationship of blood glucose against insulin requirement in a 15 year old human insulin dependent diabetic patient, and
- **Figure 6**: shows a plot of porcine c-peptide in response to IV glucose in the subject of Figure 5.

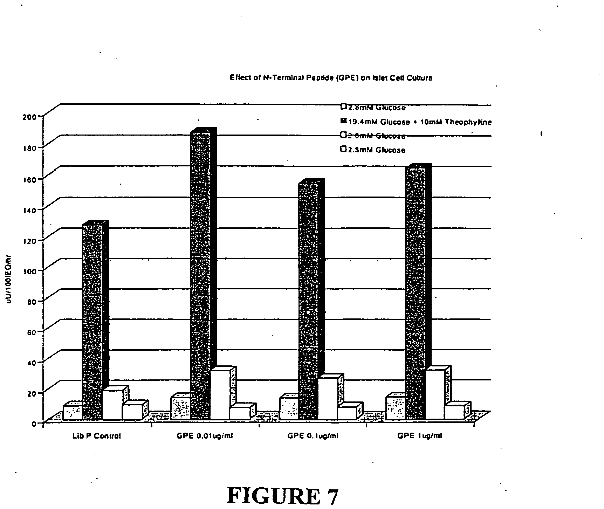

- **Figure 7**: shows the results of exposure of neonatal porcine islets in culture with GPE in comparison with control cells.

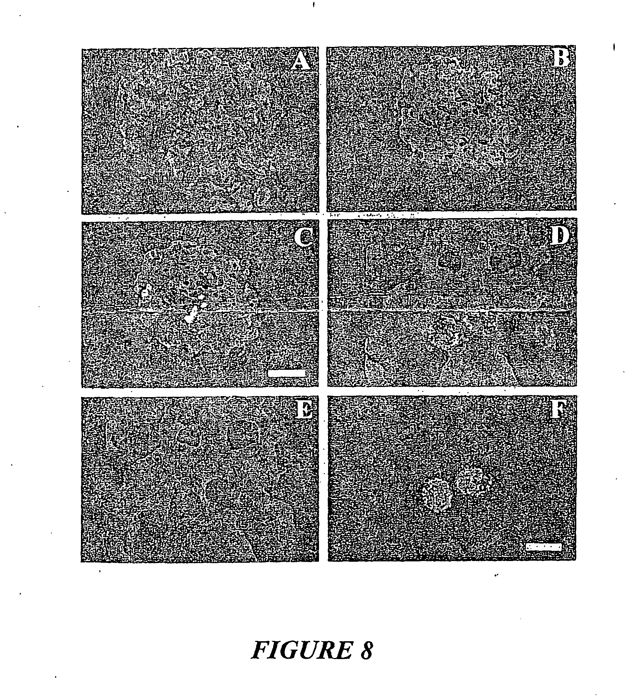

- **Figure 8**: Double labelling immunofluorescence imaged by confocal microscopy. For this set of experiments we used stacks of 6 optical sections taken at the equatorial plane of pancreatic islets. A-B : insulin detection alone (the lighter shades); in B all the cells were stained with bright fluorescence intensity, whereas in A (islets not cultured in the presence of SC) only few cells were brilliant and the majority of them was either poorly or unstained. C: double fluorescence image for insulin (the mid-range shades) and BrdU (the lightest shade). This labelling can be detected in the areas corresponding to the nuclear region unstained by the insulin. D: for a better identification BrdU fluorescence was displayed alone. Six BrdU positive nuclei were shown. E-F: detail of C, corresponding to the central islet area with a couple of BrdU stained cells. In E both fluorescence were shown, with BrdU labelling corresponding to two nuclear areas. In F insulin immunofluorescence was imaged alone and the negative areas corresponding to nuclei were clearly distinguishable. The bleached square in the centre of the islet (B- C) corresponded to the scanning area of the detail shown in E and F, that had been acquired first and was then followed by the scan of the entire islet. Bar for A-D: 10 µm. Bar for E-F: 2 µm.tc
- **Figure 9**: Insulin release under static glucose incubation on day 3 (a) and day 9 (b). In both instances insulin release was significantly higher (*p<0.05) only in the group where Islets were co-cultured with 400,000 SC.
- **Figure 10**: Shows two view of the subcutaneous device of one preferred form of the invention.

### DETAILED DESCRIPTION OF THE INVENTION

### 1. General

The present invention recognizes the ability to source appropriate islets from piglets which have similar structural similarities of insulin to humans, and similar physiological glucose levels to humans. The piglets used are at or near full term gestation. The islets are converted into an appropriate xenotransplantable source of islets with viability in a human being by following certain procedures in respect of the harvesting and extraction of the islets, the treatment of the islets prior to xenotransplantation as well as regimes of use of such islets.

The major advantage of porcine islet cell transplantation over human islet cell transplantation is that the islet cell source can be readily expanded, and the biosafety of the cells can be thoroughly explored prior to transplantation. From a practical viewpoint, pancreas removal and islet cell isolation can be performed expeditiously in an ideal environment.

Important considerations relevant to the use of porcine islet cells in transplantation approaches for type 1 diabetes include the following:
- The structural and biological similarities of porcine and human insulin
- The fact that porcine insulin has been used to treat diabetes for several decades (and has only been replaced by human sequence insulin relatively recently); and
- The similarity of physiological glucose levels in pigs and humans. (Weir & Bonner-Weir 1997). This effectively means that pig islet cells can be expected to react similarly to their human counterparts in maintaining equivalent blood glucose concentrations.

### 2. The nature of disease causing diabetes

Successful long-term allotransplantation of human islets can be achieved in over 80% of patients when the disease is caused by non-immune processes. In contrast, even islets obtained from a non-diabetic twin cannot reverse autoimmune diabetes long-term in the diabetic twin member. This emphasises the critical role of autoimmunity in the failure of islet transplantation. This observation has been validated in allotransplantation of rodents with diabetes caused by autoimmunity as compared with diabetes due to pancreatectomy or chemical cell destruction. No large animal model of autoimmune diabetes exists.It is possible that the use of islets from different species (xenotransplantation) could avoid autoimmune destruction of transplanted islets, as the immune process of xenotransplant rejection is different to that of allotransplant rejection, but this is entirely hypothetical in humans.

### 3. Isolation and Preparation of Porcine Islet Cells for Xenotransplantation

### 3a. Animal Source and Transportation

All animals intended as a source of pancreatic tissue for xenotransplantation are obtained from a specific pathogen-free (SPF) pig breeding facility which is maintained in accordance with the American Association for Accreditation of Laboratory Animal Care (AAALAC). The facility maintains a high-health status colony with excellent standards of husbandry, and operates a record system that is readily accessible and archived indefinitely. Donor sows and sires are selected with the underlying objective of producing strong heterosis in donor litters.

### 3b. Isolation and Purification of Islet Cells

Following surgical removal, the donor pancreases are transferred to a cleanroom facility for further processing in a cold plastic container in 50ml tubes containing cold Hanks' Balanced Salt Solution (HBSS) with 0.2% human serum
- albumin (HSA) added. Blood samples from each donor are sent for virology testing and
- toxoplasma serology. Samples from each organ are kept in a freezer at -80°C for future testing if necessary.

### 3c. Digestion

The islet cells are isolated by standard collagenase digestion of the minced pancreas via the procedure documented by Ricordi et al. (1990), though with some modifications. Using aseptic technique, the glands are distended with Liberase™ (1.5mg/ml), trimmed of excess fat, blood vessels and connective tissue, minced, and digested at 37°C in a shaking water bath for 15 minutes at 120 rpm. The digestion is achieved using lignocaine mixed with the Liberase™ solution to avoid cell damage during digestion. Following the digestion process, the cells are passed through a sterile 400mm mesh into a sterile beaker. A second digestion process is used for any undigested tissue.

We have determined that much greater yields per neonatal pig pancreas can be obtained using either pig or human Liberase™ (eg; sourced in New Zealand from Roche) rather than collagenase. Whilst there is disclosure in "Improved Pig Islet Yield and Post-Culture Recovery Using Liberase P1 Purified Enzyme Blend", T J Cavanagh et al. Transplantation Proceedings 30, 367 (1998) and in *"*Significant Progress In Porcine Islets Mass Isolation Utilizing Liberase HI For Enzymatic Low-Temperature Pancreas Digestion", H. Brandhorst et al. Transplantation Vol 68, 355-361 No. 3, August 15, 1999 the yields therefore therein are low compared to those we have discovered. If, for example, in following the procedure of Brandhorst et al. there is a yield increase of islets over collagenase of from 400 to say 800 with the procedure using human Liberase™ (ie; Liberase™ HI) as in the Brandhorst et al. procedure but confined to neonatal porcine islets such as those as 7 days post delivery extra ordinarily larger yields are possible, namely, the equivalent to from 400 which would be the case with crude collagenase to 30000 which as can be seen as very much greater than that to be expected from following the procedure of Brandhorst et al. with pigs.

### 3d. Washing and Culture

The digested tissue is washed three times, and seeded into cell culture media RPMI 1640 to which is added 2% human serum albumin (HSA), 10 mmol/L nicotinamide, and antibiotic (Ciproxin).

### 3e. Quality Control Procedures

To exclude any contamination of the tissue, quality control procedures are undertaken on cell culture samples after isolation and before encapsulation (further details are given in SOP P101). Three days after isolation, the cell culture is tested for microbiological contamination by accredited laboratories. Testing for porcine endogenous retrovirus (PERV) is undertaken in our laboratory. The *islet yield* is determined via dithizone (DTZ) staining of the cells, as specified in SOP Q200. Dithizone is a zinc-chelating agent and a supravital stain that selectively stains zinc in the islets of Langherhans, producing a distinctive red appearance.

The *viability* of the islet cells is determined using acridin orange and propidium iodide, as specified in SOP Q201. Acridin orange is a fluorescent stain that readily passes through all cell membranes to stain the cytoplasm and nucleus. Bright green fluorescence in both the nucleus and cytoplasm on exposure to ultraviolet (UV) light denotes intact live cells. Conversely, propidium iodide is a fluorescent stain that cannot pass through an intact membrane. It emits a bright red fluorescence when exposed to UV light, and the presence of propidium iodide in a cell nucleus indicates severe damage or a dead cell.

### 3f. Determination of in vitro Insulin Secretory Capacity

Static glucose stimulation (SGS) is used to assess *in vitro* function of the porcine islets by exposing them to low and high concentrations of glucose and theophylline. Determination of the *in vitro* insulin secretory capacity is undertaken on both free islets (after 3 days in culture) and after their subsequent encapsulation or confinement.

### 4. Xenotransplantation

### 4a. The viability of the islets for xenotransplantation

The processes by which islets are purified prior to transplantation are traumatic to these highly specialised tissues. Such trauma can induce necrosis or apoptosis - the latter being quite delayed.

Further trauma may result from encapsulation. Processes used by us in both the preparation of islets and their encapsulation have been optimised to ensure minimal damage to the islets. Such procedures have ensured zero warm ischaemia (compared with hours with most human islet preparations), have involved the use of nicotinamide to enhance successful *in vitro* explanation, have involved minimal incubation time with collagenase or liberase, have involved swift non-traumatic encapsulation technology, have involved the use of IgF-1 (or the GPE tripeptide thereof), the use of an anaesthetic such as lignocaine, and the use of an antibiotic such as ciproxin etc.

Our preferred preparation preferably uses neonatal (7-day old) islets which is crucial in both limiting islet trauma during purification, and assuring sufficient maturation of the islets for stimulated insulin production.

The IgF-1 (Human Insulin-like Growth Factor I) is used in order to induce immature porcine islets to mature to their insulin-producing form. IgF-1 is a potent mitogenic growth factor that mediates the growth promoting activities of growth hormone postnatally. Both IgF-1 and IgF-2 are expressed in many cell types and may have endocrine, autocrine and paracrine functions. The preferred form of IgF-1 we have found to be the amino-teminal tripeptide glycine-proline-glutamate of IgF-1 (GPE).

### 4b Vascularised Subcutaneous collagen tube procedure.

The islets may be implanted in a suitably vascularised subcutaneous collagen tube using Sertoli cells as the method of preventing tissue immune rejection.

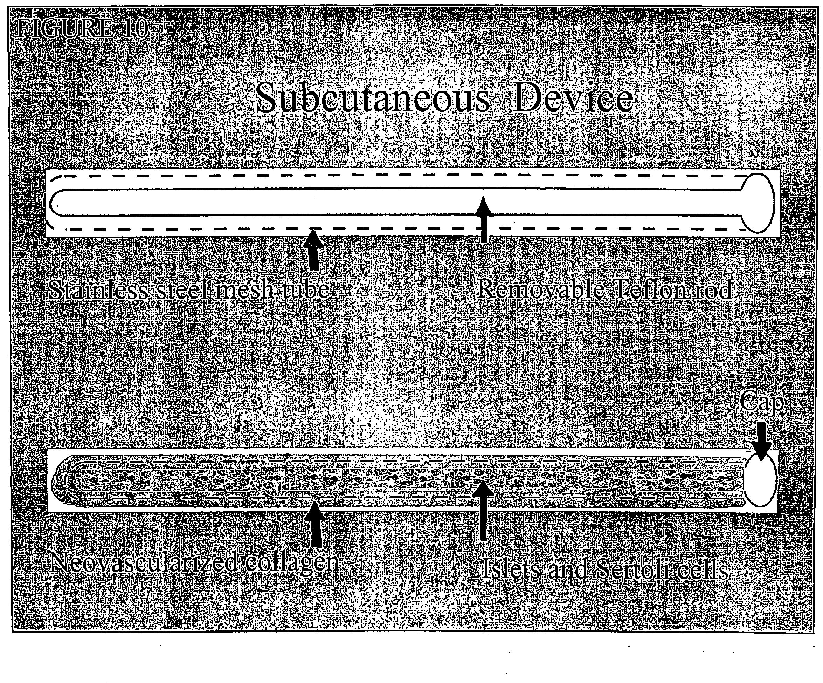

Figure 10 illustrates view of this device.

In brief a closed ended tube of stainless steel mesh containing a loosely fitting Teflon rod is inserted subcutaneously in the intended graft recipient. Six weeks later the rod is removed-leaving a highly vascularised tube of collagen. A mixture of islets prepared as above together with Sertoli cells prepared by the method of Rajotte from the testes of the same piglets from whom the islets were obtained. The Sertoli cells are mixed with the islets in a ratio of about 10 to 1,000 cells/islet and inserted into the vascular tube. Which is then sealed with a Teflon stopper.

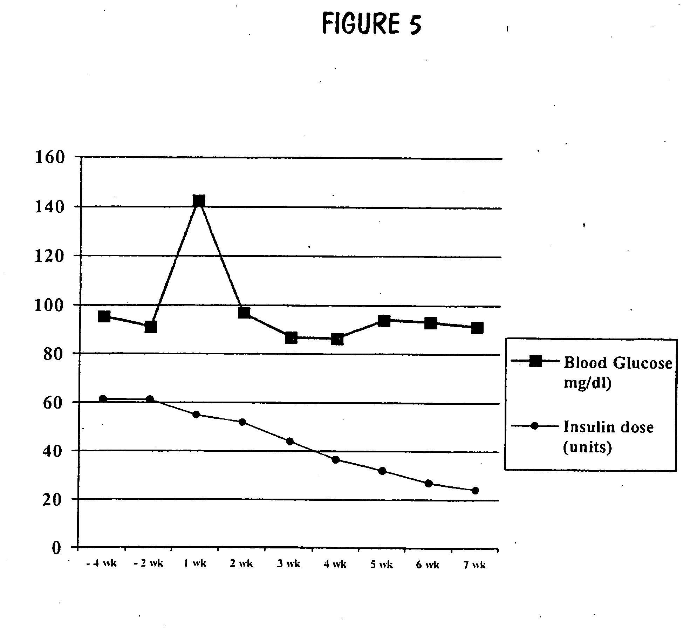

The effect of such a device containing piglet islets (approx 300,000) +Sertoli cells on insulin requirement in a 15 year old human insulin dependent diabetic subject is shown in Figure 5.

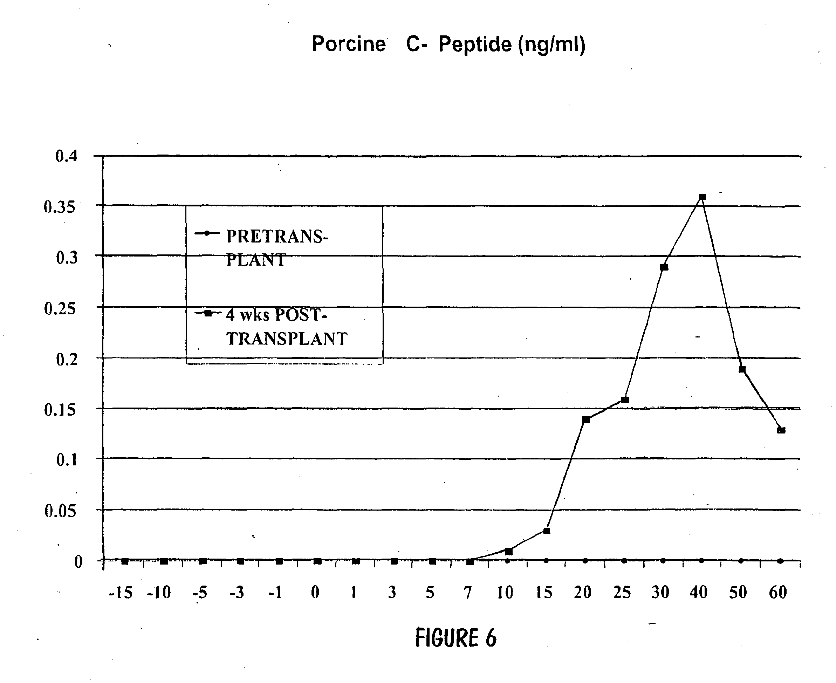

The secretion of porcine C-peptide in response to IV glucose in this subject 4 weeks after transplantation is shown in the Figure 6 below.

### 4c. Alginate Encapsulation Procedure

Sodium alginate used for this procedure is extracted from raw material sources (seaweed) and prepared in a powdered ultrapure form. The sterile sodium alginate solution (1.6%) is then utilised at the Diatranz Islet Transplant Centre to manufacture encapsulated islets. The encapsulation procedure (University of Perugia) involves extruding a mixture of islets and sodium alginate solution (1.6%) through a droplet generating needle into a bath of gelling cations (calcium chloride). The islets entrapped in the calcium-alginate gel are then coated with positively charged poly-L-ornithine followed by an outer coat of alginate (0.05%). The central core of alginate is then liquefied by the addition of sodium citrate. Most capsules contain 3 islet cells and have a diameter of 300 to 400µm.

The encapsulated islets are kept in cell culture, and then checked for contamination, insulin release and viability before transplantation. They are only released for transplantation if all quality control tests are negative.

### 4d. Drugs used in the recipient

Transplantation does not require and avoids the need for cytotoxic agents to suppress the immune system. Such agents are able to enter the alginate microcapsule and cause islet toxicity, as well as causing systemic toxicity. Instead, nicotinamide and a special diet are used.

The transplantation procedures of our earlier patent specification have the ability over a period prior to rejection of providing porcine insulin. In this respect, we ourselves conducted clinical trials.

Four type 1 diabetic adolescents received 10,000 free islets/kg bodyweight by intraperitoneal injection. The islets were located from term piglets using the standard collagenase digestion, purification and culture techniques described in section 3.2. All four recipients received oral nicotinamide (1.5 g/day) and a casein-free as herein defined diet both pre- and post-transplantation. A prompt reduction in insulin requirements, which was not clearly dose-related, was noted in the first week after transplantation. The reduction in insulin dosage range from 21 to 32%, and the response lasted for up to 14 weeks. However, insulin doses subsequently returned to their previous levels.

The most likely reason for the transplant failure in these patients was chronic rejection. However, no adverse effects were noted.

We have shown alginate encapsulation is a suitable method for limiting such chronic rejection. We have shown alginate-encapsulated porcine islet cell transplants in two human diabetic patients, prolonged functioning of the transplants. The islets were transplanted by intraperitoneal injection, one patient receiving 15,000 IEQ/kg (total 1,300,000 islets) and the other 10,000 IEQ/kg (total 930,000 islets). Both patients were treated pre- and post-transplantation with oral nicotinamide and a soy-based/casein-free as herein defined diet.

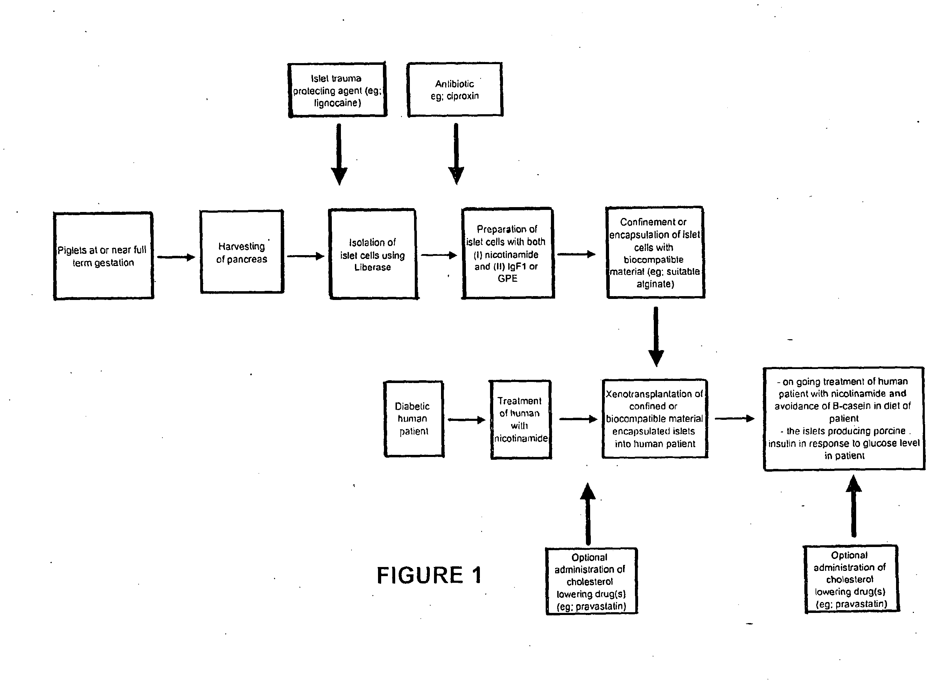

The preferred procedure as shown in Figure 1 was used for the preparation, the encapsulation being as aforesaid. Islet cells of -7 days to +10 days full gestation were used.

### 4e. Use of Sertoli Cells

The present invention is directed to dealing with the islet transplant mass problem. Islet -cells originally derive from both differentiation of pancreatic exocrine duct stem cells, and replication of pre-existing, already differentiated cells. The -cell proliferation activity varies upon age (from 10% of the fetal period to 3% of adulthood), although specific factors or conditions may influence and/or promote growth of these cells at any time, and in particular :
1) glucose;
2) pregnancy;
3) hormones, such as prolactin and growth hormone;
4) growth factors such as -cellulin, vasculo-endothelial growth factor, platelet-derived growth factor, and insulin-like growth factors I and II (IGF I, II).

However, it is quite unlikely that, within a transplant setting, under putative unfavourable environmental conditions, the -cells undergo significant replication. The latter has been observed in alginate-microencapsulated islets, although the death rate clearly surpassed the islet cell mitotic rate, thereby leading to progressive loss of the original transplanted cell mass.

To deal with this problem, ancillary "nursing" cells, associated with cell growth induction capacity might result in prolongation of the islet -cells survival and functional competence.

In the present invention, Sertoli cells have been employed as a potential "nursing" cell system for the islets. Sertoli cell functional properties, and in particular synthesis of growth factors such as IGF-I, transforming growth factor , endothelial growth factor, and clusterin, favour the -cell expansion, while on the immunity front, expression of Fas-L provides the islet transplant with immunoprotection.

In accordance with the invention Sertoli cells upon in vitro co-culture with homologous, isolated whole rat pancreatic islets, could alter the -cell low proliferative cycle, by reversing the adult elements into foetal-like status has been examined. This would coincide with significantly enhanced -cells mitogenicity.

In the specific instance of Sertoli Cells, production of several different growth factors is coupled to expression of molecules, such as Fas-L, that are associated with immune properties.

Theoretically, Sertoli Cell-derived Fas-L could induce apoptotic destruction of the tissue transplant-directed CD4⁺ and CD8⁺ Tc populations. Furthermore, Sertoli Cells in their original anatomic situation, are part of the so called "hemato-testicular barrier". Therefore the Sertoli Cell-related immunoprivileges might be possibly transferred to the islet cell system. Sertoli Cell-related cell growth induction capacity, in conjunction with immunomodulatory activity, have been proven to succeed in preliminary experimental studies. Cerebral grafts of Sertoli Cell relieved symptoms in rats with pharmacologically-induced hemiparkinsonism. Moreover, allograft of unpurified Sertoli Cells in combination with islets, beneath the kidney capsule of rats with streptozotocin-induced diabetes, significantly prolonged the achieved normoglycemia, as compared to controls receiving islet Transplant alone.

Our studies have shown that Sertoli Cells, at proper concentrations, are able to promote significant -cell replication when co-incubated with homologous islets. Additionally, the increase in -cell mitotic activity results in an associated significant increment in endogenous insulin output, both in standard culture conditions, and under glucose stimulation, as compared with Sertoli Cell-free control islet cells.

Our studies have demonstrated that:
a) in vitro, the induced -cell proliferation resulted in increased endogenous insulin output, both in basal and after glucose stimulation.
b) transferral of our in vitro results to an in vivo transplant system, where the achieved reversal of hyperglycaemia, in mice with STZ-induced diabetes, has been prolonged by microcapsules that contained (Islets +Sertoli Cell) compared with (Islets) alone. Optimisation of the ideal Sertoli Cell:islet mass ratio would maximize Sertoli Cell beneficial effects on islets.

### 5. Examples

### 5a. Reference Examples of use of IgF-1

Porcine islets in culture which were exposed to IgF-1, increased their insulin response to glucose, by up to a 3-fold increase.

| | **Incubated 24hrs with 0.1ug/ml IgF-1 after isolation** | **CONTROL- no IgF-1** |
|---|---|---|
| Insulin secretion In response to 19.4mM Glucose +10mM Theophyline After 3 days | 236uU/hr/100IEQ | 75.2uU/hr/100IEQ |

A concentration of 0.1ug/ml IgF-1 in culture is sufficient to produce optimal insulin secretion during glucose challenge. No further benefit was achieved by increasing the concentration of IgF-1.

| | **Incubated 24hrs with 0.1 ug/ml** | **Incubated 24hrs with 1.0 ug/ml IgF-1** |
|---|---|---|
| Insulin secretion In response to 19.4mM Glucose +10mM Theophyline After 3 days | 58uU/hr/100IEQ | 56.8uU/hr/100IEQ |

Variations on the duration of IgF-1 exposure were tried on the porcine islet cells. However no increased benefit was found on culturing the islets with IgF-1 beyond a 24hrs period, post isolation.

| | **Incubated 7 days with 0.1ug/ml IgF-1** | **Incubated 24hrs with 1.0 ug/ml IgF-1** |
|---|---|---|
| Insulin secretion In response to 19.4mM Glucose +10mM Theophyline 7days post isolation | 58uU/hr/100IEQ | 57.5uU/hr/100IEQ |

This increased insulin production persisted to 14 days post IgF-1 exposure. Longer durations are yet to be investigated.

| | **14 days** | **3 days** |
|---|---|---|
| | **post IgF-1 Exposure** | **post IgF-1 Exposure** |
| Insulin secretion In response to 19.4mM Glucose + 10mM Theophyline | 1.3-fold increase Compared to control | 1.5-fold increase Compared to control |

Withdrawal of Nicotinamide from the culture media eliminated the benefit of IgF-1 on islet insulin production.

| | **Incubated 3 days With 0.1ug/ml IgF-1 Without Nicotinamide** | **Incubated 3 days With culture Media Without Nicotinamide** |
|---|---|---|
| Insulin secretion In response to 19.4mM Glucose +10mM Theophyline After 3 days culture Post isolation | 47.6uU/hr/100IEQ | 55.9uU/hr/100IEQ |

A concentration of 0.1ug/ml IgF-2 during culturing appeared to increase insulin production of porcine islet cells, after an initial exposure of 24 hrs. However, this increase was transient to 3 days post exposure.

| | **Incubated 24hrs with 0.1ug/ml IgF-2 day 1.** | **Control** |
|---|---|---|
| Insulin secretion In response to 19.4mM Glucose +10mM TheophylineAfter 3 days culture Post isolation | 105.8/100IEQ | 75.2r/100IEQ |

| | **Incubated 24hrs With 0.1ug/ml IgF-2 day 1.** | **Control** |
|---|---|---|
| Insulin secretion In response to 19.4mM Glucose +10mM TheophylineAfter 3 days culture Post isolation | 32uU/hr/100IEQ | 39.8 uU/hr/100IEQ |

Prolonged exposure to IgF-2 beyond 24hrs, failed to increase the insulin production of the islet cells in response to glucose.

| | **Incubated 24hrs With 0.1ug/ml IgF-2 day 1.** | **Control** |
|---|---|---|
| Insulin secretion In response to 19.4mM Glucose +10mM TheophylineAfter 3 days culture Post isolation | 105.8/100IEQ | 75.2r/100IEQ |

| | **Incubated 7 days With 0.1ug/ml IgF-2** | **Control** |
|---|---|---|
| Insulin secretion In response to 19.4mM Glucose +10mM TheophylineAfter 7 days culture Post isolation | 38.4uU/hr/100IEQ | 39.8uU/hr/100IEQ |

### 5b. Reference Example : Effect of N-Terminal Tripeptide (GPE) of Insulin like growth factor (IGF-1) on the function of neonatal porcine islet cells.

GPE is a tripeptide (gly-pro-glu) derived from IGF-1. It is a novel neuroactive peptide with a potent effect on acetylcholine and dopamine release in cortical slices. The studies done using GPE support the concept that the proteolytic products of the IGF-1 precursor play a role in the regulation of brain functions.

The aim of this example was to present the effect of GPE on the function of isolated porcine islets in vitro.

### Method

- Islet cell isolation with 2 pancreases;
- Isolation following the previously discussed protocol;
- RPMI media added with Ciproxin, nicotinamide, Human serum albumin
- IGF1- Long R 3 IGF-1 Media Grade, GroPep (AU100), stock solution 100 ug/ml (in 10 mM HCl): dilute further in RPMI medium to the final concentrations: 1ug/ml (1: 100), 0.5 ug/ml (1: 200) and 0.1 ug/ml (1:1000).
- GPE, IGF1 (1-3), Bachem AG, Lot No.0538925, stock solution of 100 ug/ml (in water):
   dilute further in RPMI medium to the final concentrations: 1ug/ml (1:100), 0.1 ug/ml (1:1000) and 0.01 ug/ml (1:10000)
      1. IGF10.1ug/ml
      2. IGF1 0.5ug/ml
      3. IGF1 1.0ug/ml
      4. GPE 0.0 1ug/ml
      5. GPE 0.1 ug/ml
      6. GPE 1.0ug/ml

Keep the cells 3 days in culture before Static Glucose Stimulation (SGS). SGS involves exposure of the cells to low and high concentration of glucose to check insulin production. Using 0.1 ug/ml concentration add IGF1 and GPE to two plates 24 hours before SGS (day 2 after isolation)

### Results

Exposure of neonatal porcine islets in culture to GPE increased the insulin response to glucose up to 11.5 fold compared with the control cells.(Stimulation Index control 13.3 compared to 24.8 when GPE was used) Viability of the cells was >85% DTZ, AO/PI staining)

A concentration of 0.01ug/ml of GPE in culture is sufficient to produce optimal response during glucose challenge. No further benefit was achieved by increasing the concentration of GPE in culture. See figure below.

The results suggest that GPE could be used during porcine islet cell culture to improve the quality and function of the cells before transplantation. Furthermore GPE is a novel neuroactive peptide found in human brain.

### 5c. Examples of the effect of lidocaine when used during porcine pancreatic digestion, on islet yield and viability.

Lidocaine is a membrane stabiliser and phospholipase A2 inhibitor. When used at a 1mM concentration during Collagenase digestion of 7d old porcine pancreas, a 2-fold increase in islet yield is produced.

Islet endocrine function was assessed after 3 days in culture via static glucose stimulation. Islets isolated with Lidocaine during digestion produced a 3-fold increase in insulin secretion in response to glucose challenge.

| | **Collagenase alone** | **Collagenase + 1mM Lidocaine** |
|---|---|---|
| Average islet yield | 40,960 IEQ/g | 88,183 IEQ/g |

| | **Collagenase alone** | **Collagenase + 1mM Lidocaine** |
|---|---|---|
| Insulin secretion in response to 19.4mM Glucose +10mM Theophyline After 3 days culture Post isolation | 46.4 uU/hr/100IEQ | 163.8 uU/hr/100IEQ |

Conclusion: The use of Lidocaine during pancreatic digestion increases the insulin production/g of pancreas by 6-fold.

### 5d. Reference Examples of the effects of Ciproxin on Islet function as assessed by static glucose stimulation.

Freshly prepared neonatal pig islets were prepared by standard isolation procedure and cultured for two days in RPMI medium with standard additions.

Streptomycin (100mg/ml)and Penicillin (100U/ml) were included in one flask and Ciproxin (3 mcg/ml) in another.

The islets were harvested and an aliquot subjected to stimulation with theophylline and high glucose.

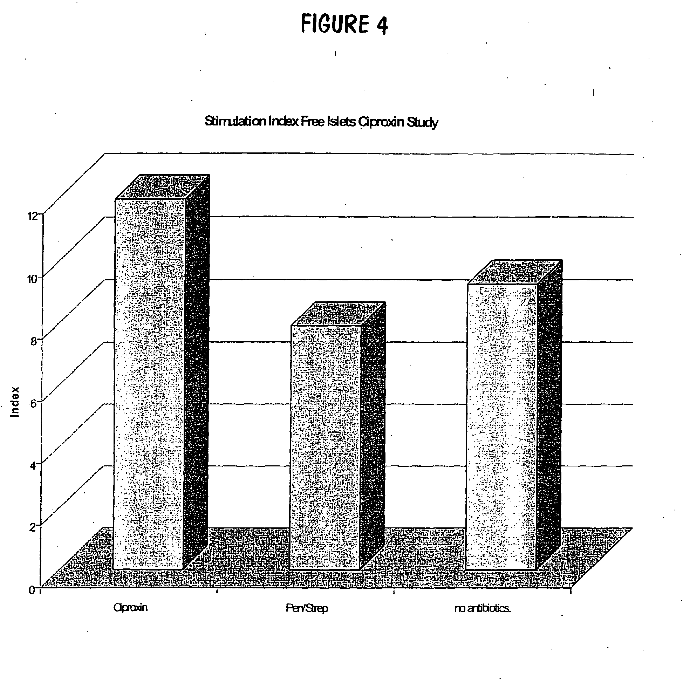

The comparative insulin release from the islets---a measure of viability is shown in the Figure 4 below.

### 5e. Reference Examples of the effects of collagenase from various sources on islet yield and function

Pancreases of neonatal piglets aged 7 days were obtained as above and islets extracted by the same process, varying only the source and amount of collagenase. The yield/gram of pancreas is shown in the Figure.

Islets extracted using these variations in collagenase source and amount were assessed for viability using propidium iodide and dithizone for insulin content.
DTZ staining >85%
AO/PI >85%

The islets were then assessed for functionality by static glucose stimulation as above. The results are shown in the Figure below.

It is apparent that the Liberase™ preparations at suitable concentrations gave higher yields and function in vitro than the previously optimised concentration of Collagenase P.

### 5f. Reference Examples of the comparative effectiveness of islets prepared with Liberase P or H in vivo

Islets prepared with the best concentration of Liberase P and H in this way were injected intraperitoneally into CD1 mice made diabetic by intravenous streptozotocin. The dose used was 10 islets/g body weight of mouse. Ten days after such treatment the number of mice no longer diabetic was assessed.

1/7 of the mice treated with the islets isolated with Liberase P and 4/7 of those islated with Liberase H were non diabetic.

Similar experiments were performed using spontaneously diabetic NOD mice. Of the surviving mice at 10 days after transplantation 3/7 of the Liberase P treated islets and 3/3 of the Liberase H islets were no longer diabetic

### 5g. Reference Example of Islet Encapsulation Procedure

The encapsulation procedure has been developed by Dr R Calafiore at the University of Perugia. The novel medium size microcapsules (300-400µ MSM) are prepared by atomizing the islet-alginate suspension through a special microdroplet generator.

Sodium alginate used for this procedure is extracted form raw material sources (seaweed) and prepared in powdered ultrapure form (Keltone LVCR).

The encapsulation procedure involves extruding a mixture of islets and sodium alginate solution (1.6%) through a droplet generating needle into a bath of gelling cations (calcium chloride). The islets entrapped in the calcium-alginate gel are then coated with positively charged poly-L-ornithine followed by an outer coast of alginate (0.05%). The central core of alginate is then liquefied by the addition of sodium citrate. Most capsules contain 3 islet cells and have a diameter of 300 to 400µm.

The encapsulated islets are kept in cell culture, and then checked for contamination, insulin release and viability before transplantation.

### 5h. Reference Example of Sertoli Cell Coculturing

The following examples outline specific materials, methods and procedures which have been employed. As would be known by those skilled in the art, other suitable techniques or materials, or variations on the following techniques or materials may be employed .

### A) BASIC METHODOLOGIES

### 1) Pancreatic islet source and isolation procedure

Islets were isolated and purified from adult Sprague Dawley male rats, weighing approximately 150 g., according to the following method:
Upon laparotomy, the common bile duct was cannulated by a polyethylene catheter, and ligated prior to its merging into the small bowel. A collagenase solution was manually delivered into the duct so as to induce retrograde distention of the pancreas. The distended pancreas was gently detached from the small and large bowel, and finally the stomach, so as to avoid any discontinuation of the organ capsule, which would cause collagenase's leakage and pancreas deflation, with compromission of the digestion process. Upon removal of the spleen and lymphonodes, the pancreas was placed in 37 C bath, and shaken (130-140 cycles/min.) until the tissue came finely apart. The tissue digest, upon wash in Hank's balanced salt solution (HBSS) was centrifuged against discontinuous Eurocollins + Ficoll density gradients, at 400 g for 25 min. at 4 C. A pure islet cell band was detected and recovered at the 1.096/1.060 density interface. The average absolute yield in islet equivalents (IEQ, islets quoted as they all measured 150µm in diameter) per pancreas was 600.

### 2) Sertoli Cell source and retrieval procedure

Sertoli Cells were separated from the testes of prepubertal (15 days old) SD rats, according to the following adaptation of prior art methods.

Each testicle pair was stored, soon after retrieval, in cold Eurocollins for 30 minutes. Under sterile conditions, the testes were finely minced until a fine homogeneous tissue was obtained. The tissue underwent multiple-step enzymatic digestion by collagenase P, trypsin, and DNAase. The digest, after several washes in TCM 199, was resuspended in HAM F12 supplemented with nicotinamide, 3-isobutyl-1-methylxanthine, serum albumin and pen-strep. The tissue suspension was finally filtered through 500µm pore-size stainless mesh, plated in 100x15 mm. Petri dishes and culture-maintained at 37 C in 5% air/CO2 for 48 hours (HAM F-10, supplemented with Nicotinamide, IBMX, 10% Fetal Bovine Serum, 110 mg/dl glucose).

Finally, the tissue was treated with 20 mM tris-(hydroxymethyl)-aminomethane hydrochloride buffer to eliminate any residual germinal cells. Final yield of the procedure was 60 millions of 90% pure Sertoli Cell per testicle. Sertoli Cell morphological characterization and identification was conducted by staining the preparation with Sudan III, and anti-vimentin MoAb, while cell viability was documented by Trypan Blue exclusion staining (the cells were incubated for 15 minutes at room temperature, in the presence of 0.2% Trypan Blue in HBSS). Additionally, cell viability was also assessed by staining with ethidium bromide and fluorescein diacetate, under fluorescence microscopy. Only the Sertoli Cell preparations that scored over 90% viability entered the study protocols. At the end of the separation process, to detect eventual contaminating Leydig cells, the tissue suspension was cyto-enzymatically stained to assess the activity of 3- hydroxy-steroidodehydrogenase (staining with Nitro-Blue Tetrazolium), an enzyme that is specifically associated with this cell type. Presence of Leydig cells was negligible and they completely disappeared by 48 hrs. of culture maintenance. Last, Sertoli Cell functional competence was shown by the in vitro capability of these cells to convert testosterone enanthate into 17- estradiol in presence of follicle-stimulating hormone : the assay's specificity was granted by the fact that FSH receptors are selectively expressed by Sertoli Cells. All hormones were measured by R.I.A. (intra-assay c.v. = 3.2-4.5%%; inter-assay c.v. =4-8%)

### B) IN VITRO STUDIES

### 1) Islet-Sertoli Cell co-incubation

Batches of 20 islets were incubated with either 200000 (islet +Sertoli Cell 200000) or 400000 Sertoli Cell (islet +Sertoli Cell 400000), in HAM F-12, in quadruplicate. Isolated islets or Sertoli Cell batches served as controls. Tissue culture medium was changed every 3 days and the supernatant was stored at -20 C. At 11 days of co-culture, the study was terminated, and the samples were processed for examination under confocal laser scanning microscopy (CLSM). Insulin (IRI) levels were determined by RIA (itra-assay c.v. <5.5%; inter-assay c.v.=9.0%). To assess the islet functional performance, the batches were sequentially exposed to glucose at different concentrations (50-300-50 mg/dl) in a 2 hr. static incubation system.

### 2) Microscopic studies

### a) sample processing

The cell preparations were treated following adaptations of prior art procedures:
Bromo-deoxyuridine (BrdU) was added to the culture medium to a final concentration of 10µM, for 24 hours of culture. Subsequently, the islets were washed with PBS and fixed with 4% paraformaldehyde in 0.1 M phosphate buffer (pH 7.0) for 30 min. at room temperature. The BrdU-labelled islets, upon denaturation of DNA by acid hydrolysis (2M HCl), and following washes, were incubated with a 1:25 dilution of a mouse MoAb anti-BrdU (anti-BrdU mouse MoAb) in PBS with 0.3% Triton X-100 (PBS/I) for 18 hours on a rotating plate, at room temperature. After wash, the islets were then incubated with a 1:25 dilution of fluorescein isothiocyanate (FITC)-conjugated goat anti-mouse immunoglobulin G in PBS/T for 18 hours on a rotating plate. After wash, the islets were stored in PBS/T and thereafter prepared (aqueous mounting, immuno-Fluore mounting medium) for CLSM examination. For double labelling experiments the islets were finally incubated with mouse anti-insulin Ab for 18 hours at 4 C, and with anti-mouse-TRITC, for 18 hours at 4 C, as a secondary Ab.

### b) Confocal Laser Microscopy (CLSM) Examination

Samples were imaged by a LSM410 microscope. This confocal system was coupled with a 25mW multiline Argon ion laser and a 1 mW HeNe ion laser as light sources, used to reveal FITC signals with 488 nm, and TRITC signal with a 543 nm wavelength, respectively. Samples were examined with a x100, 1.3 numerical aperture PlanNeofluar objective lens. Images were acquired, frame by frame, with a scanning mode format of 512x512 pixels. The HeNe laser, which produces a major line at 543 nm, was employed for double labelling experiments. To this end, FITC and TRITC signals were separated by a secondary dichroic mirror (565 nm) and simultaneously detected by two photomultiplier tubes. A band pass filter (520±15 nm for FITC) and a barrier filter (590 nm for TRITC) were placed before the two photomultiplier tubes to avoid overlapping between the two signals, as described in the prior art.

### c) Image processing analysis

Digitalized optical sections, ie Z series of confocal data ("stacks") were transferred from the CLSM to the graphics workstation Indigo Irix XS24 and stored on the graphics workstation with a scanning mode format of 512x512 pixels and 2567 grey levels. The image processing was performed using the ImageSpace software (Molecular Dynamics), as previously described. ,

To reduce the unwanted background noise generated by the photomultiplier signal amplification, all the image stacks were treated with a three-dimensional filter (Gaussian filtering) that was carried out on each voxel, with a mask of 3 pixel in the x, y and z direction (3x3x3). The FITC and TRITC or PI signals were elaborated to optimize the contrast, the brightness and the intensity of the images.

To analyse the colocalization of BrdU and insulin, an ImageSpace software tool that creates a two-dimensional scatter plot diagram was employed. It shows in a third colour how dual labels are spatially distributed or co-localized. In the co-localization scatter plot it has been detected a cluster corresponding to the areas of the specimen recorded by both detectors. Within this cluster we have selected the area with high pixel values, to avoid background interference. The resulting image could be superimposed on both fluorescence or transmitted (phase contrast) images. For a better visualization we have employed a phase contrast image to show cell topography and the sites of colocalization in green. Photographs were taken by a digital video recorder Focus ImageCorder Plus using 100 ASA Tmax black and white film or 100 ASA Kodacolor Gold film.

To assess positivity of the BrdU-labelled nuclei, at least 40 fluorescent brilliant spots corresponding to DNA replicon clusters were counted, independently of the exhibited fluorescent morphology. To assess the percentage of replicating cells, all the insulin-labelled cells were counted, by assembling the optical sections from confocal microscopy analysis, through the entire islet. Finally, the BrdU-labelled cells were related to the islet cells they belonged to. Confocal laser microscopy analysis excluded that all cells were counted more than once.

### C) IN VIVO STUDIES

The same Sertoli Cell and islet tissue batches employed for the in vitro studies were used to graft CD-1 mice, weighing approximately 25g, that had been rendered diabetic by low dose, multiple injections of streptozotocin (STZ), at 40 mg/kg/d, for five days.

Blood glucose levels were measured at 10 days of the last STZ injection, in post-absorptive conditions. Only mice that were associated with blood glucose values in excess of 400 mg/dl during at least 3 consecutive measurements entered the study. "Transplant survival" was associated with reversal of hyperglycaemia and maintenance of blood glucose lower than 150 mg/dl. Animals that returned to hyperglycaemia after the initial remission were assumed to fail. 10 diabetic mice were divided into two groups :
1) n=5 received intraperitoneal transplant of a unit dose of 1000 islets + 10000 Sertoli Cell containing alginate/poly-L-ornithine (AG/PLO) microcapsules;
2) n=5 control mice received intraperitoneal transplant of 1000 islets alone, containing AG/PLO microcapsules.

Blood glucose was measured by a reflectometer at 3 and 7 days of transplant, and thereafter once a week throughout the study.

The microcapsules were prepared according to our method where islet containing AG gel beads were coated with PLO and an outer layer of AG, so as to provide the islets with an immunoisolatory shield.

### D. STATISTICAL ANALYSIS

### Confocal microscopy studies

Data were the mean of three different experiments and were expressed as mean ± SD. The asterisk indicates significant differences (p<0.01) in a Student's paired t test. All of the other differences were found to be not significant with p>0.01:

### In vitro metabolic studies

i) Insulin release during static glucose incubation : data were analysed by repeated measures of the analysis of variance (ANOVA) followed by a post-hoc test (Bonferroni's procedure). Data were the mean ± SD and p<0.05 were significant.
ii) Conversion of testosterone into estradiol : data were expressed as mean ± SD. The asterisk designates significant differences in a Student's paired t test.

### E. RESULTS

### 1) Assessment of cell mitotic activity upon co-culture with Sertoli Cells

With reference to Figures 8A and 8B, when the islets were cultured alone, only a few cells showed brilliant fluorescence pattern (the lighter areas of Figure 8A) corresponding to the insulin immunolabelling, whereas the majority of the cells was poorly or not stained at all. On the contrary, all the islet cells belonging to the islet/Sertoli Cell co-culture groups stained positively for insulin as shown by very bright fluorescence patterns (the lighter areas of Figure 8B).

The presence of replicating cells within the islets with high insulin expression was examined using double labelling experiments with CLSM to obtain on the same focal plane, within the same cell, the fluorescence derived from insulin and from BrdU

As illustrated in Figure 8C, in the equatorial region of the islet, some BrdU positive cells, whose cytoplasms were all strongly stained by the anti-insulin antibody, were detected. When the two images were merged, these areas coincided with the nuclear region (Figure 8, C-E), that looked as a dark, unstained region when insulin alone was displayed (Figure 8, B-F).

### 2) Quantification of -cell mitotic figures

To assess the percentage of pancreatic -cells entering the S-phase of the cell cycle upon Sertoli Cell co-culture, we used labelling with 5'-BrdU, followed by fluorescent immunostaining. The results of these experiments are shown in Table 1.

**Table 1**

| Percentage of -cells that were positive to immunostaining for 5'-BrdU, with or w/o incubation with SC, at 12 days of in vitro culture. For each experiment, 300 cells were counted. Data were expressed as mean±SD from three separate experiments. *p values <0.01 were significant. **IMMUNOSTAINING WITH BrdU** | | |
|---|---|---|
| **SERTOLI CELLS (SC)** | **% VIABLE (SC) CELLS.** | **BrdU-POSITIVE** |
| None | 91±5 | 1±1 |
| 200000 | 93±3 | 5.8±1.3* |
| 400000 | 92±4 | 8.1±1.* |

Cell proliferation was assessed at 24 hours after mitogenic stimulation, since it had previously been reported that this is the time, after a mitogenic stimulus, when the highest number of cells, that have entered S-phase, is detectable. While under control conditions no more than 1% of 5'-BrdU positive -cells were evidenced, the percentage of the -cell-related mitotic figures significantly raised from 1% to 8.1% upon islet cell-Sertoli Cell co-incubation.

### 3) In vitro metabolic data

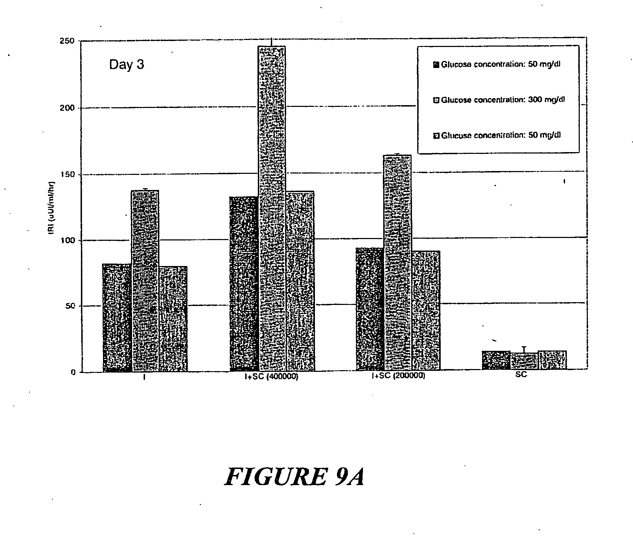

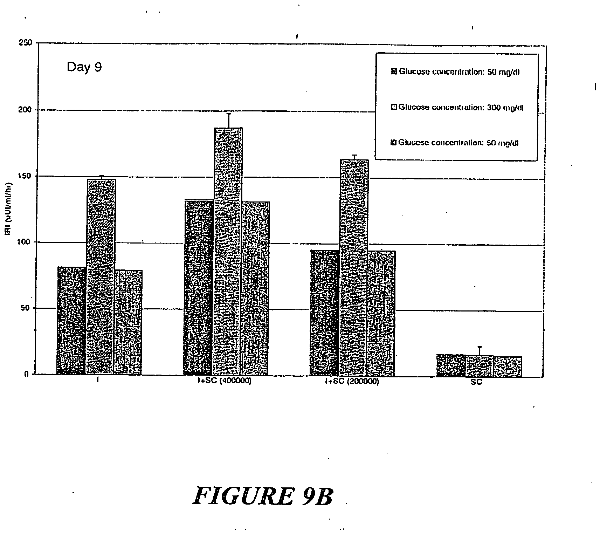

Consistent with the above reported morphological findings, we have observed significant increase in endogenous insulin output from the islets that were co-cultured with Sertoli Cells, in comparison with either islets or Sertoli Cells alone, throughout the time period of in vitro culture maintenance (total IRI levels from 90±10 µU/ml - islets alone - to 127.5±12 µU/ml - islets +Sertoli Cells, p<0.05). The enhanced insulin secretion, deriving from the islet-Sertoli Cell complexes, as compared to I alone, under static glucose stimulation, on both days 3 (Figure 9a) and 9 (Figure 9b) of culture maintenance, statistical significance was reached only by the I+ 400k Sertoli Cell combination.

### 4) In vivo transplant data

Reversal of hyperglycaemia was fully achieved in both experimental groups and sustained in 100% of the recipients, at 45 days post-transplant, while at 52 days of graft one animal of the group (Islet), against none of the (Islet +Sertoli Cell) group failed. The first animal belonging to the (I+Sertoli Cell) group showed recurrence of hyperglycaemia at day 75 post-transplant. At that time, 3/5 animals of the group (Islet) had failed. At day 100 of graft, three animals of group (Sertoli Cell+Islet) against one from group (Islet) were still euglycemic.

### REFERENCES

1. Brelje TC, Scharp DW, Lacy PE, Ogren L, Talamantes F, Robertson M, Friesen HG and Sorenson RL. Effect of homologous placental lactogens, prolactins and growth hormones on islets -cells division and insulin secretion in rat, and human islets: implications for placental lactogen regulation of islet function during pregnancy. Endocrinology 1993; 132: 879-887.
2. Hayek A, Beattie GM, Cirulli V, Lopez AD, Ricordi C and Rubin JS. Growth Factor/Matrix-Induced Proliferation of Human Adult -Cells. Diabetes 1995; 44: 1458-1460.
3. Ramiya VK, Maraist M, Arfors KE, Schatz DA, Peck AB, and Cornelius JG. Reversal of insulin-dependent diabetes using islets generated in vitro from pancreatic stem cells. Nature Med, 2000; 6, 3: 278-282.
4. Huotari, MA, Palgi J, Otonkoski T. Growth factor-mediated proliferation and differentiation of insulin-producing INS-1 and RINm5F cells: identification of -cellulin as a novel -cell mitogen Endocrinology 1998; 139:1494-1499.
5. Sharpe RM. Intratesticular control of steroidogenesis. Clin Endocrinol (Oxf) 1990;33,787-807.
6. Buch JP, Lamb DJ, Lipshultz LI and Smith RG. Partial characterization of a unique growth factor secreted by human Sertoli cells. Fertil Steril 1988; 49: 658-665
7. Jègou B. The Sertoli cell. Bailliere's Clinical and Metabolism 1992; 6, 273-311.
8. Saporta S, Cameron DF, Borlongan CV and Sanberg PR. Survival of rat and porcine Sertoli cell transplant in the rat striatum without cyclosporine-A immunosuppression. Exp Neurol 1997;146, 299-304.
9. Sanberg PR, Borlongan CV, Othberg AI, Saporta S, Freeman TB and Cameron DF. Testisderived Sertoli cells have a trophic effect on dopamine neurons and alleviate hemiparkinsonism. Nat Med 1997; 3: 1129-1132.
10. Korbutt GS, Elliott JF and Rajotte RV. Cotransplantation of Allogeneic Islets With Allogeneic Testicular Cell Aggregates allows Long-Term Graft Survival Without Systemic Immunosuppression. Diabetes 1997;46, 317-322.
11. Mather JP and Philips DM. Primary culture of testicular somatic cells. In: Methods for Serum Free Culture of Cells of the Endocrine System, DW Barnes, editors. New York; Liss, 1984.p. 24-25.
12. Le Magueresse B and Jègou B. In vitro effect of germ cells on the secretory activity of Sertoli cells recovered from rats of different ages. Endocrinology 1988; 22: 1672-1680.
13. Galdieri M, Ziparo E, Palombi F, Russo MA and Stefanini MJ. Pure Sertoli cell cultures: a new model for the study of somatic-germ cell interaction. J Androl 1981; 5: 249-259.
14. Brelje TC, Sorenson RL. Role of prolactin versus growth hormone on islets -cell proliferation in vitro: implications for pregnancy. Endocrinology 1991; 128: 45-57.
15. Neri LM, Raymond Y, Giordano A, Capitani S and Martelli AM. Lamin a is a part of the internal nucleoskeleton of human erythroleukemia cells. J Cell Physiol 1999; 178: 284-295.
16. Neri LM, Martelli AM and Maraldi NM. Redistribution of DNA topoisomerase II after in vitro stabilization of human erythroleukemic nuclei by heat or Cu++ revealed by confocal microscopy. Mic Res Techn 1999; 36: 179-187.

## Claims

1. A method of preparing a xenotransplantable porcine islet preparation, capable upon xenotransplantation of producing porcine insulin in an appropriate recipient mammal, the method including or comprising:
(i) harvesting the pancreas of piglets at from -20 to +20 days of full term gestation; and
(ii) extracting islets from a culture of the harvested pancreas using a suitable collagenase;
the culture of the harvested pancreas being -
(a) of mechanically reduced harvested pancreas, and
(b) a supportive mammalian albumin substantially free of non-human microbiological agents;
wherein the pancreas and/or islets are subject to the trauma protecting agent lignocaine, and wherein the islets are mixed with and/or co-cultured with Sertoli cells.

2. A method as claimed in claim 1 wherein:
(a) the islets (at least at some stage in the performance of the method) are exposed to nicotinamide;
(b) the mammalian albumin is human serum albumin (HSA); and/or
(c) the collagenase is selected from human Liberase® and porcine Liberase®.

3. A method as claimed in claim 1 or 2 wherein the piglets are at -7 to +10 days of full term gestation.

4. A method as claimed in any one of the preceding claims wherein said Liberase® is human Liberase®.

5. A method as claimed in any one of the preceding claims wherein the islets are treated with nicotinamide after their extraction from the pancreas.

6. A method as claimed in any one of the preceding claims wherein the method includes the further step of treating the islets with IgF-1 or the N-terminal tripeptide of IgF-1 (GPE).

7. A method as claimed in any one of the preceding claims wherein the preparation or isolation of the Sertoli cells includes use of, or exposure to, a buffer to selectively reduce germinal cells.

8. A method as claimed in claim 7 wherein the buffer is tris- (hydroxymethyl)-aminomethane hydrochloride.

9. A method as claimed in any one of the preceding claims wherein the ratio of Sertoli cell to islets employed is substantially 10-1,000 Sertoli cells/islet.

10. A xenotransplantable porcine islet preparation prepared according to the method of one or more of claims 1 to 9.

11. An implantable device comprising the porcine islet preparation as claimed in claim 10.

12. An implantable device as claimed in claim 11, wherein the device is a vascularised subcutaneous tube comprising the xenotransplantable porcine islet preparation, which is capable of producing porcine insulin in response to glucose within a recipient mammal,
the tube *in vivo* limiting the egress of the islet cells and allowing an abundant access to blood vessels, the tube also employing or containing Sertoli cells to substantially reduce or prevent tissue immune rejection within the recipient mammal.

13. An implantable device as claimed in claim 12 wherein the islet cells have been isolated from a culture supported by HSA using a suitable collagenase and prior to containment in the tube have been exposed to nicotinamide.

14. An implantable device as claimed in claim 12 or 13 wherein the Sertoli cells are mixed with the islets prior to insertion into, or within, the tube.

15. An implantable device as claimed in any one of claims 12 to 14 wherein the Sertoli cells are mixed with the islets substantially in a ratio of 10-1,000 cells/islet.

16. An implantable device as claimed in any one of claims 12 to 15 wherein said culture has been of mechanically reduced harvested pancreatic tissue, such tissue having been exposed to a trauma reducing agent.

17. An implantable device as claimed in claim 11, wherein the device is a xenotransplantable capsule comprising the xenotransplantable porcine islet preparation, which is capable of producing porcine insulin in response to glucose within a recipient mammal within its biocompatible encapsulating material or materials, the capsule being such that the encapsulation is such as to prevent tissue contact with said islet cell(s) by tissue of a recipient mammal yet *in vivo* will allow glucose entry to the islet cells and the egress of porcine insulin from such islet cells, wherein the islet cells are associated with Sertoli cells.

18. An implantable device as claimed in claim 17 wherein the islet cells have been isolated from a culture supported by HSA using a suitable collagenase and prior to encapsulation have been exposed to nicotinamide.

19. An implantable device as claimed in claim 17 or 18 wherein said culture has been of mechanically reduced harvested pancreatic tissue, such tissue having been exposed to a trauma reducing agent.

20. An implantable device as claimed in any one of claims 17 to 19 wherein said encapsulation has been of islet cells in the presence of a suitable antibiotic.

21. An implantable device as claimed in any one of claims 17 to 20 wherein the Sertoli cells are mixed with the islets prior to encapsulation.

22. A implantable device as claimed in claims 17 to 21 wherein the Sertoli cells are co-cultured with the islet cells prior to encapsulation.

23. An implantable device as claimed in any one of claims 17 to 22 wherein the Sertoli cells: islet ratio is 10-1,000 cells : islet.

24. An implantable device as claimed in any one of claims 17 to 22 wherein the biocompatible suitable material is a suitable alginate.

25. An implantable device as claimed in any one of claims 11 to 24 for use in the treatment of a mammalian patient predisposed to or suffering from diabetes.

26. An implantable device for use according to claim 25 wherein there is included also the step of administering nicotinamide to the recipient mammal prior to or after the implantation step.

27. An implantable device for use according to claim 25 or claim 26 wherein the method further includes the step of prescribing for the patient, prior to or after the implantation step, a casein-free diet.

28. An implantable device for use according to claim 25 or claim 26 wherein the method further includes the step of subjecting the patient prior to or after the implantation step to a cholesterol lower drug regime.

## Patentansprüche

1. Verfahren zur Herstellung einer xenotransplantierbaren Präparation von Langerhans-Inseln von Schweinen, welche auf die Xenotransplantation hin zur Erzeugung von Schweine-Insulin in einem geeigneten Empfänger-Säuger fähig ist, welches Verfahren einbezieht oder umfasst:
(i) Entnehmen der Bauchspeicheldrüse (Pankreas) von Ferkeln bei von -20 bis +20 Tagen der normalen Trächtigkeitsdauer; und
(ii) Extrahieren der Langerhans-Inseln aus einer Kultur der entnommenen Bauchspeicheldrüse unter Verwendung einer geeigneten Collagenase; wobei die Kultur der entnommenen Bauchspeicheldrüse von folgendem ist:
(a) einer mechanisch reduzierten entnommenen Bauchspeicheldrüse,
und
(b) einem unterstützenden Säugeralbumin, das im wesentlichen frei
von nicht-humanen mikrobiologischen Agenzien ist;
wobei die Bauchspeicheldrüse und/oder Langerhans-Inseln dem vor Trauma schützenden Agens Lignocain ausgesetzt sind, und wobei die Langerhans-Inseln gemischt werden und/oder gleichzeitig kultiviert werden mit Sertoli-Zellen.

2. Verfahren nach Anspruch 1, wobei:
(a) die Langerhans-Inseln (zumindest in irgendeinem Stadium der Durchführung des Verfahrens) Nicotinamid ausgesetzt werden;
(b) das Säugeralbumin humanes Serumalbumin (HSA) ist; und/oder
(c) die Collagenase ausgewählt ist aus humaner Liberase® und porziner Liberase®.

3. Verfahren nach Anspruch 1 oder 2, wobei sich die Ferkel bei -7 bis +10 Tagen der normalen Trächtigkeitsdauer befinden.

4. Verfahren nach jedem der vorangehenden Ansprüche, wobei die Liberase® humane Liberase® ist.

5. Verfahren nach jedem der vorangehenden Ansprüche, wobei die Langerhans-Inseln nach ihrer Extraktion aus der Bauchspeicheldrüse mit Nicotinamid behandelt werden.

6. Verfahren nach jedem der vorangehenden Ansprüche, wobei das Verfahren den weiteren Schritt des Behandelns der Langerhans-Inseln mit IgF-1 oder dem N-terminalen Tripeptid von IgF-1 (GPE) umfasst.

7. Verfahren nach jedem der vorangehenden Ansprüche, wobei die Präparation oder Isolation der Sertoli-Zellen die Verwendung von einem Puffer oder das Aussetzen daran umfasst, um selektiv Keimzellen zu reduzieren.

8. Verfahren nach Anspruch 7, wobei der Puffer Tris(hydroxymethyl)-aminomethanhydrochlorid ist.

9. Verfahren nach jedem der vorangehenden Ansprüche, wobei das Verhältnis von verwendeten Sertoli-Zellen zu Langerhans-Inseln im wesentlichen 10-1.000 Sertoli-Zellen/Langerhans-Insel beträgt.

10. Xenotransplantierbare Präparation von porzinen Langerhans-Inseln gemäß dem Verfahren nach einem oder mehreren der Ansprüche 1 bis 9.

11. Implantierbare Vorrichtung, umfassend die Präparation der porzinen Langerhans-Inseln nach Anspruch 10.

12. Implantierbare Vorrichtung nach Anspruch 11, wobei die Vorrichtung ein vaskularisierter subkutaner Schlauch ist, umfassend die xenotransplantierbare Präparation der porzinen Langerhans-Inseln, welche zur Erzeugung von Schweine-Insulin als Reaktion auf die Glukose innerhalb eines Empfänger-Säugers fähig ist,
wobei der Schlauch *in vivo* den Austritt von Inselzellen beschränkt und einen völlig zureichenden Zugang von Blutgefäßen erlaubt, wobei der Schlauch außerdem Sertoli-Zellen verwendet oder enthält, um eine Immunabstoßung von Gewebe innerhalb des Empfänger-Säugers wesentlich zu vermindern oder zu verhindern.

13. Implantierbare Vorrichtung nach Anspruch 12, wobei die Inselzellen aus einer durch HSA unterstützten Kultur unter Verwendung einer geeigneten Collagenase isoliert worden sind und vor dem Einschluss in den Schlauch Nicotinamid ausgesetzt worden sind.

14. Implantierbare Vorrichtung nach Anspruch 12 oder 13, wobei die Sertoli-Zellen mit den Langerhans-Inseln vor der Einführung in den Schlauch, oder innerhalb des Schlauchs, gemischt werden.

15. Implantierbare Vorrichtung nach jedem der Ansprüche 12 bis 14, wobei die Sertoli-Zellen mit den Langerhans-Inseln im wesentlichen in einem Verhältnis von 10-1.000 Zellen/Langerhans-Insel gemischt werden.

16. Implantierbare Vorrichtung nach jedem der Ansprüche 12 bis 15, wobei die Kultur aus mechanisch reduziertem entnommenen
Bauchspeicheldrüsengewebe angelegt worden ist, welches Gewebe einem Trauma-reduzierendem Agens ausgesetzt worden ist.

17. Implantierbare Vorrichtung nach Anspruch 11, wobei die Vorrichtung eine xenotransplantierbare Kapsel ist, umfassend die xenotransplantierbare Präparation von porzinen Langerhans-Inseln, welche zur Erzeugung von Schweine-Insulin als Reaktion auf die Glukose innerhalb eines Empfänger-Säugers innerhalb ihres biokompatiblen einkapselnden Materials oder Materialien fähig ist, wobei die Kapsel eine solche ist, bei der die Einkapselung derart ist, dass der Gewebekontakt mit der/den Inselzelle(n) durch das Gewebe eines Empfänger-Säugers verhindert wird, sie jedoch *in vivo* den Zugang von Glukose zu den Inselzellen und den Austritt von Schweine-Insulin aus den Inselzellen zulassen wird, wobei die Inselzellen mit Sertoli-Zellen assoziiert sind.

18. Implantierbare Vorrichtung nach Anspruch 17, wobei die Inselzellen aus einer durch HSA unterstützten Kultur unter Verwendung einer geeigneten Collagenase isoliert worden sind und vor der Einkapselung Nicotinamid ausgesetzt worden sind.

19. Implantierbare Vorrichtung nach Anspruch 17 oder 18, wobei die Kultur aus mechanisch reduziertem entnommenen Bauchspeicheldrüsengewebe angelegt worden ist, welches Gewebe einem Trauma-reduzierendem Agens ausgesetzt worden ist.

20. Implantierbare Vorrichtung nach jedem der Ansprüche 17 bis 19, wobei die Einkapselung in Inselzellen in der Gegenwart eines geeigneten Antibiotikums bestanden hat.

21. Implantierbare Vorrichtung nach jedem der Ansprüche 17 bis 20, wobei die Sertoli-Zellen mit den Langerhans-Inseln vor der Einkapselung gemischt werden.

22. Implantierbare Vorrichtung nach Ansprüchen 17 bis 21, wobei die Sertoli-Zellen gemeinsam mit den Inselzellen vor der Einkapselung kultiviert werden.

23. Implantierbare Vorrichtung nach jedem der Ansprüche 17 bis 22, wobei das Verhältnis von Sertoli-Zellen zu Langerhans-Inseln 10-1.000 Zellen/Insel beträgt.

24. Implantierbare Vorrichtung nach jedem der Ansprüche 17 bis 22, wobei das biokompatible geeignete Material ein geeignetes Alginat ist.

25. Implantierbare Vorrichtung nach jedem der Ansprüche 11 bis 24, zur Verwendung in der Behandlung eines Säuger-Patienten, der für Diabetes prädisponiert ist oder daran leidet.

26. Implantierbare Vorrichtung zur Verwendung nach Anspruch 25, wobei außerdem der Schritt des Verabreichens von Nicotinamid an den Empfänger-Säuger vor oder nach dem Implantationsschritt einbezogen ist.

27. Implantierbare Vorrichtung zur Verwendung nach Anspruch 25 oder Anspruch 26, wobei das Verfahren weiterhin den Schritt des Verschreibens an den Patienten, vor oder nach dem Implantationsschritt, einer Casein-freien Diät umfasst.

28. Implantierbare Vorrichtung zur Verwendung nach Anspruch 25 oder Anspruch 26, wobei das Verfahren weiterhin den Schritt des Unterziehens des Patienten, vor oder nach dem Implantationsschritt, einem Cholesterin-senkenden Arzneiregime umfasst.

## Revendications

1. Méthode de préparation d'une préparation d'îlots porcins xénotransplantable, capable après xénotransplantable de produire de l'insuline porcine chez un mammifère receveur approprié, la méthode incluant ou comprenant :
(i) le prélèvement des pancréas de porcelets d'âge compris entre -20 et +20 jours du terme de gestation ; et
(ii) l'extraction d'îlots à partir d'une culture des pancréas prélevés en utilisant une collagénase appropriée ;
la culture des pancréas prélevés étant constituée -
(a) de pancréas prélevés réduits mécaniquement, et
(b) d'une albumine de mammifère servant de support, sensiblement exempte d'agents microbiologiques non humains ;
dans laquelle les pancréas et/ou les îlots sont soumis à la lignocaïne, agent de protection contre les traumatismes, et dans laquelle les îlots sont mélangés avec et/ou co-cultivés avec des cellules de Sertoli.

2. Méthode telle que revendiquée dans la revendication 1 dans laquelle :
(a) les îlots (au moins à un certain stade de réalisation de la méthode) sont exposés à une nicotinamide ;
(b) l'albumine de mammifère est une albumine-sérique humaine HSA ; et/ou
(c) la collagénase est choisie parmi la Libérase^{®} humaine et la Libérase^{®} porcine.

3. Méthode telle que revendiquée à la revendication 1 ou 2 dans laquelle les porcelets sont âgés de -7 à + 10 jours du terme de gestation.

4. Méthode telle que revendiquée dans l'une quelconque des revendications précédentes dans laquelle ladite Libérase^{®} est une Libérase^{®} humaine.

5. Méthode telle que revendiquée dans l'une quelconque des revendications précédentes dans laquelle les îlots sont traités avec une nicotinamide après leur extraction à partir des pancréas.

6. Méthode telle que revendiquée dans l'une quelconque des revendications précédentes dans laquelle la méthode comprend l'étape supplémentaire de traitement des îlots avec IgF-1 ou le tripeptide N-terminal de IgF-1 (GPE).

7. Méthode telle que revendiquée dans l'une quelconque des revendications précédentes dans laquelle la préparation ou l'isolement des cellules de Sertoli comprend l'utilisation de, ou l'exposition à, un tampon pour réduire sélectivement les cellules germinales.

8. Méthode telle que revendiquée dans la revendication 7 dans laquelle le tampon est du chlorhydrate de tris-(hydroxyméthyl)-aminométhane.

9. Méthode telle que revendiquée dans l'une quelconque des revendications précédentes dans laquelle le ratio de cellules de Sertoli par rapport aux îlots utilisé est sensiblement de 10-1 000 cellules de Sertoli/îlot.

10. Préparation d'îlots porcins xénotransplantable préparée selon la méthode de l'une ou de plusieurs des revendications 1 à 9.

11. Dispositif implantable comprenant la préparation d'îlots porcins telle que revendiquée à la revendication 10.

12. Dispositif implantable tel que revendiqué à la revendication 11, dans lequel le dispositif est un tube sous-cutané vascularisé comprenant la préparation d'îlots porcins xénotransplantable, qui est capable de produire de l'insuline porcine en réponse au glucose chez un mammifère receveur,
le tube *in vivo* limitant la libération des cellules d'îlots et permettant un accès abondant aux vaisseaux sanguins, le tube utilisant ou contenant également des cellules de Sertoli pour réduire sensiblement ou empêcher le rejet immunitaire de tissu chez le mammifère receveur.

13. Dispositif implantable tel que revendiqué à la revendication 12 dans lequel les cellules d'îlots ont été isolées à partir d'une culture, contenant de l'albumine-sérique humaine en tant que support, utilisant une collagénase appropriée et ont été exposées à la nicotinamide avant leur introduction dans le tube.

14. Dispositif implantable tel que revendiqué à la revendication 12 ou 13 dans lequel les cellules de Sertoli sont mélangées avec les îlots avant l'insertion dans le, ou à l'intérieur du, tube.

15. Dispositif implantable tel que revendiqué dans l'une quelconque des revendications 12 à 14 dans lequel les cellules de Sertoli sont mélangées avec les îlots sensiblement dans un ratio de 10-1 000 cellules/îlot.

16. Dispositif implantable tel que revendiqué dans l'une quelconque des revendications 12 à 15 dans lequel ladite culture provient d'un tissu pancréatique prélevé qui a été mécaniquement réduit, ce tissu ayant été exposé à un agent réducteur de traumatisme.

17. Dispositif implantable tel que revendiqué à la revendication 11, dans lequel le dispositif est une capsule xénotransplantable comprenant la préparation d'îlots porcins xénotransplantable, qui est capable de produire de l'insuline porcine en réponse au glucose chez un mammifère receveur dans son matériau ou ses matériaux d'encapsulation biocompatible(s), la capsule étant telle que l'encapsulation permet d'empêcher un contact tissulaire de la (les) dite(s) cellule(s) d'îlots avec un tissu d'un mammifère receveur mais qui *in vivo* permet l'entrée de glucose dans les cellules d'îlots et la libération d'insuline porcine à partir de ces cellules d'îlots, dans lequel les cellules d'îlots sont associées à des cellules de Sertoli.

18. Dispositif implantable tel que revendiqué à la revendication 17 dans lequel les cellules d'îlots ont été isolées à partir d'une culture, contenant de l'albumine-sérique humaine en tant que support, utilisant une collagénase appropriée et qui a été exposée à la nicotinamide avant encapsulation.

19. Dispositif implantable tel que revendiqué à la revendication 17 ou 18 dans lequel ladite culture provient d'un tissu pancréatique prélevé qui a été mécaniquement réduit, ce tissu ayant été exposé à un agent réducteur de traumatisme.

20. Dispositif implantable tel que revendiqué dans l'une quelconque des revendications 17 à 19 dans lequel ladite encapsulation consiste en des cellules d'îlots en présence d'un antibiotique approprié.

21. Dispositif implantable tel que revendiqué dans l'une quelconque des revendications 17 à 20 dans lequel les cellules de Sertoli sont mélangées avec les îlots avant encapsulation.

22. Dispositif implantable tel que revendiqué dans les revendications 17 à 21 dans lequel les cellules de Sertoli sont co-cultivées avec les cellules d'îlots avant encapsulation.

23. Dispositif implantable tel que revendiqué dans l'une quelconque des revendications 17 à 22 dans lequel le ratio cellules de Sertoli : îlots est de 10-1 000 cellules : îlot.

24. Dispositif implantable tel que revendiqué dans l'une quelconque des revendications 17 à 22 dans lequel le matériau biocompatible approprié est un alginate approprié.

25. Dispositif implantable tel que revendiqué dans l'une quelconque des revendications 11 à 24 utilisable dans le traitement d'un patient mammifère prédisposé au, ou souffrant de, diabète.

26. Dispositif implantable pour utilisation selon la revendication 25 qui inclut également l'étape d'administration de nicotinamide au mammifère receveur avant ou après l'étape d'implantation.

27. Dispositif implantable utilisable selon la revendication 25 ou 26 dans laquelle la méthode comprend en outre l'étape consistant à prescrire au patient, avant ou après l'étape d'implantation, un régime sans caséine.

28. Dispositif implantable utilisable selon la revendication 25 ou la revendication 26 dans laquelle la méthode comprend en outre l'étape consistant à soumettre le patient, avant ou après l'étape d'implantation, à un régime destiné à réduire le cholestérol.
